# EUROPEAN PATENT SPECIFICATION

(11) **EP 4 459 258 B1**
(45) Date of publication and mention of the grant of the patent: **11.11.2026**
(21) Application number: 21969851.1
(22) Date of filing: 31.12.2021
(51) Int. Cl.: G01N 15/14, G01N 33/49, G01N 33/50, G01N 35/10, G01N 15/01, G01N 35/00

(54) **ANIMAL RETICULOCYTE TESTING METHOD AND SAMPLE ANALYZER**
VERFAHREN ZUM TESTEN VON TIERRETIKULOZYTEN UND PROBENANALYSEGERÄT
PROCÉDÉ DE TEST DE RÉTICULOCYTES D'UN ANIMAL ET ANALYSEUR D'ÉCHANTILLON

(43) Date of publication of application: 06.11.2024
(73) Proprietor: Shenzhen Mindray Animal Medical Technology Co., Ltd., Shenzhen, Guangdong 518110 (CN)
(72) Inventor: ZHANG, Xinjun, Shenzhen, Guangdong 518110 (CN); KONG, Fangang, Shenzhen, Guangdong 518110 (CN); SHI, Tao, Shenzhen, Guangdong 518110 (CN); YANG, Zhuxiang, Shenzhen, Guangdong 518110 (CN); WANG, Shengxi, Shenzhen, Guangdong 518110 (CN)
(74) Representative: KIPA AB
(86) International application number: PCT/CN2021/144050
(87) International publication number: WO 2023/123508

(56) References cited:
- CN-A- 101 336 290
- CN-A- 103 439 523
- CN-A- 110 018 299
- CN-A- 110 082 265
- CN-A- 111 829 940
- CN-U- 212 780 382
- JP-A- 2000 310 642
- US-A1- 2002 037 589
- US-A1- 2021 041 341

## Description

### TECHNICAL FIELD

The disclosure relates to the field of medical device technologies, and more specifically, to an animal reticulocyte testing method and a sample analyzer.

### BACKGROUND

A sample analyzer may be used to test animal blood (venous blood or arterial blood) samples, e.g., measure relevant parameters of red blood cells, white blood cells, platelets, hemoglobin, reticulocytes, etc. in blood samples. The inventors have found through research that the accuracy of current sample analyzers in testing results of animal reticulocytes is unstable, that is, reticulocyte testing results of some species of animals are relatively accurate, but reticulocyte testing results of some species of animals are not accurate enough.

JP 2000 310642 A discloses a system for analysing biological samples of a plurality of types of animals under analysis conditions suitable for the animal species.

### SUMMARY

In view of this, the disclosure provides an animal reticulocyte testing method and a sample analyzer, for ensuring the accuracy of reticulocyte testing results of various different types of animals and improving the wide applicability of the animal reticulocyte testing method.

The present application is set out in the appended set of claims.

It can be learned from the above technical solutions that, after collecting the blood sample of the animal, the sample analyzer provided in the embodiments of the disclosure may determine the reticulocyte incubation time corresponding to the animal based on the animal type of the animal, and control an incubation time of the blood sample based on the reticulocyte incubation time, to prepare the sample fluid to be tested. Because a fragmentation status of reticulocytes in the sample fluid to be tested that is obtained based on this reticulocyte incubation time is within a preset reasonable range, the accuracy of a testing result of the reticulocytes is ensured. It can be learned that this sample analyzer may improve the accuracy of reticulocyte testing results of various different types of animals and expand the scope of application of animal testing.

### BRIEF DESCRIPTION OF THE DRAWINGS

In order to describe the technical solutions in the embodiments of the disclosure or in the prior art more clearly, a brief introduction to the drawings required in the description of the embodiments or the prior art will be provided below. Obviously, the drawings in the following description only show some of the embodiments of the disclosure, and those of ordinary skill in the art would also have been able to obtain other drawings from these drawings without involving any inventive effort.
FIG. 1A is a block diagram of a structure of a sample analyzer;
FIG. 1B is a schematic diagram of a structure of a sample analyzer;
FIG. 2A is a schematic diagram of a sample fluid to be tested and a sheath liquid passing through a flow chamber;
FIG. 2B is a diagram of a testing principle of flow laser fluorescence staining of reticulocytes;
FIG. 2C is an example scatter distribution diagram of reticulocytes;
FIG. 3A is a flowchart for testing separate reticulocytes;
FIG. 3B is a flowchart for testing full-parameter reticulocytes;
FIG. 4 is a schematic diagram of a structure of a control apparatus;
FIG. 5A is an example diagram of a reticulocyte testing result interface;
FIG. 5B is an example diagram of an incubation time adjustment interface;
FIG. 6 is a schematic flowchart of an animal reticulocyte testing method; and
FIG. 7 is another schematic flowchart of an animal reticulocyte testing method.

### DESCRIPTION OF THE PREFERRED EMBODIMENTS

The technical solutions of the embodiments of the disclosure will be described below clearly and comprehensively in conjunction with accompanying drawings of the embodiments of the disclosure. Apparently, the embodiments described are merely some of, rather than all of, the embodiments of the disclosure. All other embodiments obtained by persons of ordinary skill in the art based on the embodiments of the disclosure without creative efforts shall fall within the scope of protection of the disclosure.

Sample analyzers can be used to analyze cells or other components in samples such as blood, body fluids, bone marrow, urine, and tissues. It is found through research that when current sample analyzers are used to test reticulocytes in animal blood, reticulocyte testing results of some animals may be normal, whereas reticulocyte testing results of some animals may be abnormal. In other words, the accuracy of the testing results of the current sample analyzers for animal reticulocytes is not stable enough. In order to solve this technical problem, the inventors study a process of a sample analyzer testing animal reticulocytes and propose a targeted solution.

To facilitate understanding of the technical solutions of the disclosure, some concepts and terms related to reticulocyte testing are first explained.

Reticulocytes are red blood cells after orthochromatic erythrocytes have lost their nuclei. The reticulocytes are slightly larger than mature red blood cells, and the cytoplasm thereof still contains residual ribosome dense granules, mitochondria, ferritin, and other basophilic substances. Such cells are between orthochromatic erythrocytes and mature red blood cells, with a survival period of about 1.3 days, and are an important indicator of bone marrow hematopoietic functionality.

Generally, reticulocyte testing results include one or more of: optical red blood cell count (RBC-O), reticulocyte percentage (RET%), reticulocyte count (RET#), reticulocyte hemoglobin content expression value (RHE), immature reticulocyte fraction (IRF), low fluorescence intensity reticulocyte ratio (LFR), medium fluorescence intensity reticulocyte ratio (MFR), high fluorescence intensity reticulocyte ratio (HFR), immature platelet fraction (IPF), and optical platelet count (PLT-O). If a reticulocyte count in reticulocyte testing results of a sample increases, it indicates that the bone marrow hematopoietic function of an animal of the sample is vigorous. This result is often seen in animals with hemorrhagic anemia and hemolytic anemia. If the reticulocyte count decreases, it indicates that the bone marrow hematopoietic function of the animal is low, and the animal may have aplastic anemia, a kidney disease, an endocrine disease, or other diseases.

The inventors have found that current sample analyzers are relatively accurate in testing results of reticulocytes in blood samples from dogs, cats, etc., but are less accurate in testing results of reticulocytes in blood samples from mice, etc. For example, Tables 1 and 2 provide a set of comparative experimental data on reticulocyte testing results of dogs and mice. Table 1 shows reticulocyte percentages (RET%) of six blood samples of dogs obtained by a sample analyzer and manual microscopic inspection, and Table 2 shows reticulocyte percentages (RET%) of six blood samples of mice obtained by the sample analyzer and manual microscopic inspection. As shown in Table 1, for the reticulocyte percentages of the six dog blood samples, the testing results of the sample analyzer (instrument inspection) and the testing results of manual microscopic inspection (human inspection) are basically the same, indicating that the testing results of the sample analyzer for dog reticulocytes are relatively accurate. However, as shown in Table 2, the reticulocyte percentage testing results of the same sample analyzer for six mouse blood samples are significantly different from the reticulocyte percentage testing results of manual microscopic inspection, and the testing results of the sample analyzer are significantly lower.

**Table 1**

| Blood sample | Instrument inspection (RET%) | Human inspection (RET%) | Instrument inspection/Hum an inspection | RBC-O/RBC-I |
|---|---|---|---|---|
| 1 | 2.01 | 2.09 | 0.96 | 0.99 |
| 2 | 2.52 | 2.43 | 1.04 | 0.98 |
| 3 | 2.42 | 2.5 | 0.97 | 1.03 |
| 4 | 5.12 | 5.24 | 0.98 | 1.02 |
| 5 | 0.44 | 0.36 | 1.22 | 1.01 |
| 6 | 1.53 | 1.58 | 0.97 | 0.97 |

**Table 2**

| Blood sample | Instrument inspection (RET%) | Human inspection (RET%) | Instrument inspection/Huma n inspection | RBC-O/RBC-I |
|---|---|---|---|---|
| 1 | 1.62 | 2.56 | 0.63 | 0.82 |
| 2 | 1.35 | 3.56 | 0.38 | 0.85 |
| 3 | 1.23 | 4.32 | 0.29 | 0.86 |
| 4 | 5.12 | 9.72 | 0.53 | 0.81 |
| 5 | 1.34 | 3.54 | 0.38 | 0.86 |
| 6 | 2.15 | 4.64 | 0.46 | 0.83 |

In view of the above problem of inaccurate testing results, the inventors studied conditions or factors that affect the accuracy of the testing results starting from a process of testing reticulocytes by the sample analyzer.

Currently, the sample analyzer adds a blood sample and a staining solution to a diluent and evenly mixes them to obtain a sample fluid to be tested. The sample fluid to be tested and a sheath liquid together pass through a flow chamber for testing. An optical pulse signal obtained by the testing is analyzed to obtain a reticulocyte testing result. The inventors believe that the lower reticulocyte testing results of the mouse blood samples in Table 2 may be due to a decrease in a number of reticulocytes in the sample fluid to be tested entering the flow chamber. After further research, it is found that the decrease in the number of reticulocytes may be related to an incubation process of the sample fluid to be tested. Because the osmotic pressure of the reticulocyte diluent is lower than that of the reticulocytes during the incubation process of the sample fluid to be tested, after the reticulocytes in the blood sample come into contact with the diluent, the reticulocytes become spherical under the action of the diluent. The spherical reticulocytes are stained under the action of the staining solution. Only the stained reticulocytes can produce optical pulse signals detectable in the flow chamber. If the incubation time is excessively long, the reticulocytes may be broken due to absorbing excessive diluent, resulting in a relatively low reticulocyte percentage in the testing results.

In order to verify the relationship between the accuracy of the reticulocyte testing results and the incubation time, the inventors adjusted the incubation time of the mouse sample fluid to be tested (from 25 seconds in Table 2 to 8 seconds), and then retested the six mouse blood samples, obtaining the reticulocyte testing results shown in Table 3. After comparing Table 2 with Table 3, after the incubation time of the mouse sample fluid to be tested was adjusted from 25 seconds to 8 seconds, it can be seen from the ratios of instrument inspection to human inspection that the accuracy of the reticulocyte testing results is significantly improved.

**Table 3**

| Blood sample | Instrument inspection (RET%) | Human inspection (RET%) | Instrument inspection/Human inspection | RBC-O/RBC-I |
|---|---|---|---|---|
| 1 | 2.43 | 2.56 | 0.95 | 1.01 |
| 2 | 3.62 | 3.56 | 1.02 | 1.02 |
| 3 | 4.23 | 4.32 | 0.98 | 0.98 |
| 4 | 9.92 | 9.72 | 1.02 | 0.97 |
| 5 | 3.31 | 3.54 | 0.94 | 1.01 |
| 6 | 4.63 | 4.64 | 0.99 | 1.02 |

In order to further verify the relationship between the accuracy of the reticulocyte testing results and the incubation time, the inventors adjusted the incubation time of the dog sample fluid to be tested (from 25 seconds in Table 1 to 8 seconds), and then retested the six dog blood samples, obtaining the reticulocyte testing results shown in Table 4. After comparing Table 1 with Table 4, after the incubation time of the dog sample fluid to be tested was adjusted from 25 seconds to 8 seconds, it can be seen from the ratios of instrument inspection to human inspection that the accuracy of the reticulocyte testing results is significantly reduced.

**Table 4**

| Blood sample | Instrument inspection (RET%) | Human inspection (RET%) | Instrument inspection/Human inspection | RBC-O/RBC-I |
|---|---|---|---|---|
| 1 | 2.01 | 2.09 | 0.96 | 0.98 |
| 2 | 1.95 | 2.43 | 0.80 | 0.99 |
| 3 | 2.12 | 2.5 | 0.85 | 1.02 |
| 4 | 3.12 | 5.24 | 0.60 | 1.01 |
| 5 | 0.24 | 0.36 | 0.67 | 1.02 |
| 6 | 1.23 | 1.58 | 0.78 | 0.99 |

Through the above research process, the inventors determined that the accuracy of the reticulocyte testing results of the sample analyzer is related to the incubation time of the sample to be tested. Specifically, different types of animals require different incubation times. On the basis of the research conclusion, the inventors improve the sample analyzer to improve the accuracy of reticulocyte testing results of the sample analyzer for different types of animals, thereby improving the wide applicability of the sample analyzer.

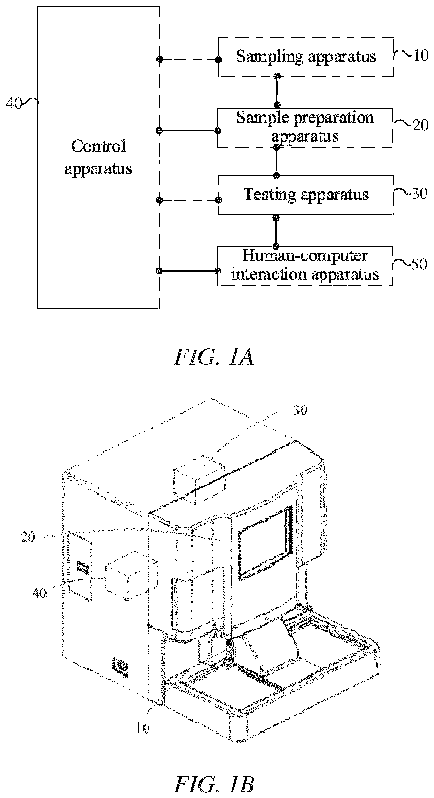

The sample analyzer provided in the embodiments of the disclosure is described below with reference to FIG. 1A to FIG. 5A.

As shown in FIG. 1A and FIG. 1B, a sample analyzer provided in an embodiment of the disclosure may include a sampling apparatus 10, a sample preparation apparatus 20, a testing apparatus 30, and a control apparatus 40.

The sampling apparatus 10 is configured to collect a blood sample of a target animal and transfer the blood sample to the sample preparation apparatus 20. The target animal may be any type of animal, such as dogs, mice, rats, cats, etc. It should be noted that in addition to analyzing blood samples, the sample analyzer can also analyze other types of samples such as urine and body fluids.

The sample preparation apparatus 20 has a reaction cell and a transfer portion. The reaction cell is configured to provide a reaction site for the blood sample to react with a treatment reagent related to reticulocyte testing, to prepare a sample fluid to be tested. The transfer portion is configured to transfer the sample fluid to be tested in the reaction cell to the testing apparatus 30. The testing apparatus 30 is configured to test the sample fluid to be tested, to obtain an optical pulse signal of cells in the sample fluid to be tested.

The sample preparation apparatus 20 may include one or more reaction cells, and different reaction cells may be used to test different blood cells. This embodiment of the disclosure is mainly used for testing reticulocytes. Therefore, the sample fluid to be tested may be prepared in a reticulocyte reaction cell. It can be understood that the treatment reagent required for preparing the sample fluid to be tested in this embodiment of the disclosure is a treatment reagent related to reticulocyte testing, for example, including a diluent and a reticulocyte staining solution. The sample preparation apparatus 20 may include a reagent supply portion to supply the treatment reagent related to reticulocyte testing. In addition, staining methods may include, but are not limited to, methylene blue staining, RNA staining, or fluorescent staining.

The sample fluid to be tested is transferred to the testing apparatus 30 by the transfer portion. In a specific implementation, the transfer portion includes a sample aspiration component such as a syringe, which can extract the sample fluid to be tested from the reaction cell and transfer the sample fluid to the testing apparatus 30 through a pipeline.

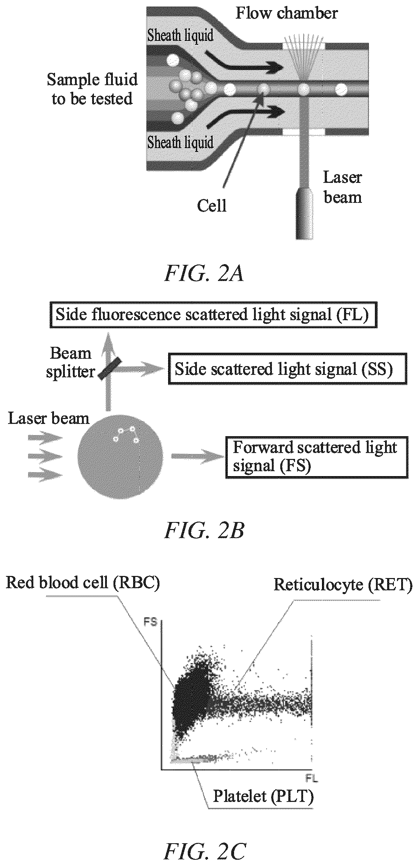

The testing apparatus 30 is specifically an optical testing apparatus, which is configured to detect an optical pulse signal of the cells in the sample fluid to be tested. The optical testing apparatus includes a laser emitter and a light detection component, as shown in a specific implementation of FIG. 2A and FIG. 2B. When the sample fluid to be tested and a sheath liquid arrive at a flow chamber together, a laser beam emitted by the laser emitter irradiates the cells passing through the flow chamber one by one. As shown in FIG. 2B, the light detection component detects optical pulse signals of the cells in a plurality of directions. Specifically, the laser beam passing through the cells forms light of two wavelengths: One is light with the same wavelength as the incident laser beam, and a scattered light signal is received by photodiodes in forward and side scattered directions; and the other is light with a longer wavelength than that of the incident laser beam, which is split by a beam splitter to separate fluorescent light, and a fluorescent scattered light signal is received by a photodiode. In this way, a forward scattered light signal (FS), a side scattered light signal (SS), and a side fluorescence scattered light signal (FL) of the cells are obtained.

After an optical detection channel detects optical pulse signals in two or more directional dimensions, the control apparatus 40 may combine optical pulse signals in any two of the plurality of directional dimensions into a two-dimensional blood cell scatter distribution diagram, for example, combine a side fluorescence scattered light signal (FL) and a side scattered light (SS) into an FL-SS diagram, combine a forward scattered light signal (FS) and a side scattered light signal (SS) into an FS-SS diagram, or combine a side fluorescence scattered light signal (FL) and a forward scattered light signal (FS) into an FL-FS diagram. In this embodiment of the disclosure, the control apparatus 40 obtains, based on the optical pulse signal of the cells in the sample fluid to be tested, a testing result of reticulocytes in the sample fluid to be tested. For example, FIG. 2C provides an example of a blood cell scatter distribution diagram, which is a blood cell scatter distribution diagram formed by a side fluorescence scattered light signal (FL) and a forward scattered light signal (FS), and the distribution diagram includes a red blood cell region, a reticulocyte region, and a platelet region. Counting the numbers of particles in the red blood cell region, the reticulocyte region, and the platelet region, testing results related to the reticulocytes, such as an optical red blood cell count, a reticulocyte percentage, a reticulocyte count, a reticulocyte hemoglobin content expression value, an immature reticulocyte fraction, a low fluorescence intensity reticulocyte ratio, a medium fluorescence intensity reticulocyte ratio, a high fluorescence intensity reticulocyte ratio, an immature platelet fraction, and an optical platelet count may be obtained.

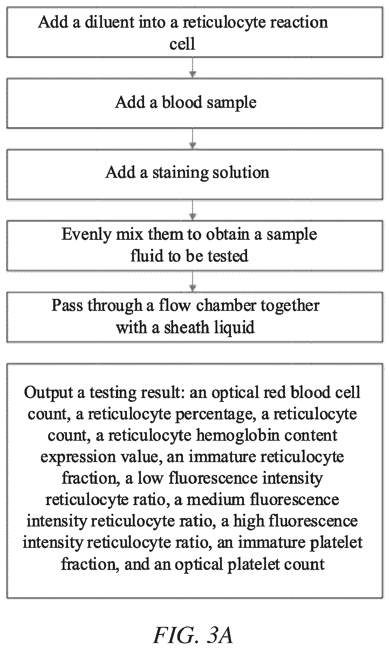

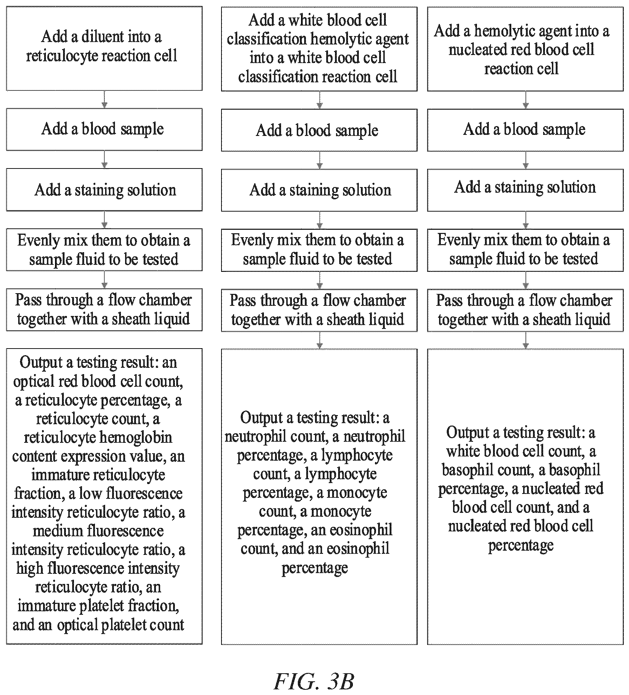

The sample analyzer may provide one or two testing processes for reticulocyte testing. For example, the sample analyzer may provide any one testing process shown in FIG. 3A or FIG. 3B, or integrate the two testing processes shown in FIG. 3A and FIG. 3B. In actual applications, users may choose to use either of them according to testing requirements.

FIG. 3A shows a reticulocyte testing process, in which a diluent, the blood sample of the target animal, and a reticulocyte staining solution are added to a reticulocyte reaction cell and evenly mixed to obtain a sample fluid to be tested. The sample fluid to be tested is transferred to the flow chamber together with a sheath liquid, and a reticulocyte testing result (including, but not limited to, an optical red blood cell count, a reticulocyte percentage, a reticulocyte count, a reticulocyte hemoglobin content expression value, an immature reticulocyte fraction, a low fluorescence intensity reticulocyte ratio, a medium fluorescence intensity reticulocyte ratio, a high fluorescence intensity reticulocyte ratio, an immature platelet fraction, and an optical platelet count) is obtained based on a testing signal of the testing apparatus.

The testing process shown in FIG. 3A may be referred to as a separate reticulocyte testing process or a separate reticulocyte testing sequence. In addition to this testing process, if the sample preparation apparatus 20 includes a plurality of reaction cells, the sample analyzer may also perform a full-parameter reticulocyte testing process (or referred to as a full-parameter reticulocyte testing sequence). The full-parameter reticulocyte testing process means that in addition to measuring reticulocyte-related parameters, related parameters of other types of cells may also be measured. A full-parameter reticulocyte testing process is shown in FIG. 3B, which may simultaneously complete testing of reticulocytes and testing of white blood cells and nucleated red blood cells. A testing process of white blood cells is: adding a white blood cell classification hemolytic agent, the blood sample of the target animal, and a staining solution into a white blood cell classification reaction cell, evenly mixing them to obtain a sample fluid to be tested, transferring the sample fluid to be tested to the flow chamber together with a sheath liquid, and obtaining a white blood cell classification result (including, but not limited to, a neutrophil count, a neutrophil percentage, a lymphocyte count, a lymphocyte percentage, a monocyte count, a monocyte percentage, an eosinophil count, and an eosinophil percentage) based on a testing signal of the testing apparatus. A testing process of nucleated red blood cells is: adding a hemolytic agent, the blood sample of the target animal, and a staining solution into a nucleated red blood cell reaction cell, evenly mixing them to obtain a sample fluid to be tested, transferring the sample fluid to be tested to the flow chamber together with a sheath liquid, and obtaining a nucleated red blood cell testing result (including, but not limited to, a white blood cell count, a basophil count, a basophil percentage, a nucleated red blood cell count, and a nucleated red blood cell percentage) based on a testing signal of the testing apparatus. It should be noted that FIG. 3B is merely an example for description. In practical applications, the testing of reticulocytes may be performed together with other types of red blood cells in a full-parameter testing process.

It can be seen from the above testing process that the blood sample of the target animal needs to be incubated in the sample preparation apparatus 20 to obtain the sample fluid to be tested. The incubation time of the sample fluid to be tested in the sample preparation apparatus may include reaction duration of the blood sample and the treatment reagent and transfer duration required for being transferred to the testing apparatus by the transfer portion. The reaction duration includes duration from the contact of the blood sample with the diluent and the staining solution to the even mixing to obtain the sample fluid to be tested, and the transfer duration includes duration for transferring the sample fluid to be tested to the flow chamber. The sample analyzer provided in this embodiment of the disclosure mainly controls incubation times of sample fluids to be tested of different types of animals by controlling either or both of the two duration. It should be noted that in the full-parameter reticulocyte testing process, the testing apparatus 30 tests all the types of cells in sequence, that is, after each reaction cell obtains its own sample fluid to be tested, the sample fluids to be tested pass through the testing apparatus 30 in sequence for testing. Therefore, although the testing is a full-parameter reticulocyte test, the incubation time of the reticulocytes may still be controlled separately. The incubation times in the full-parameter reticulocyte testing process and the separate reticulocyte testing process may be different or the same.

A specific control method of the incubation time is that the control apparatus 40 is communicatively connected to the sample preparation apparatus 20, and the control apparatus 40 performs the control process of the incubation time. In other words, the control apparatus 40 is configured to determine an animal type of the target animal, and determine a first target reticulocyte incubation time corresponding to the target animal based on the animal type of the target animal; and control an incubation time of the sample fluid to be tested in the sample preparation apparatus based on the first target reticulocyte incubation time, wherein the incubation time includes reaction duration of the sample fluid to be tested in the reaction cell and/or transfer duration of transferring the sample fluid to be tested to the testing apparatus by the transfer portion.

Specifically, the control apparatus 40 first determines the animal type of the animal body (i.e., the target animal) from which the blood sample in the sample fluid to be tested is collected, for example, the animal type includes dogs, cats, mice, rats, etc., then determines the reticulocyte incubation time corresponding to the animal type, and uses the reticulocyte incubation time as the incubation time of the sample fluid to be tested of the target animal. For ease of distinguishment from incubation times of other animals, the reticulocyte incubation time corresponding to the target animal may be referred to as a target reticulocyte incubation time. In order to distinguish the incubation time from other reticulocyte incubation times of the target animal, the incubation time may further be referred to as a first target reticulocyte incubation time (referred to as a first incubation time).

There may be a plurality of implementations for the control apparatus 40 to determine the animal type of the target animal. For example, the sample analyzer shown in FIG. 1A may further include a human-computer interaction apparatus 50. The human-computer interaction apparatus 50 may include a display apparatus such as a display screen or a touch screen, and the display apparatus may display a virtual button corresponding to at least one animal type; or the human-computer interaction apparatus 50 may include a sound apparatus to receive an indication voice of the animal type input by a user; or the human-computer interaction apparatus 50 may include a physical button corresponding to at least one animal type, etc. The animal type prompted may be an animal type preset in the sample analyzer when leaving a factory, or may be an animal type pre-defined by the user. The user inputs the indication information of the animal type to the sample analyzer through the human-computer interaction apparatus 50, for example, by touching a button corresponding to the animal type. The human-computer interaction apparatus 50 is communicatively connected to the control apparatus 40, and after receiving the animal type indication information carrying the animal type, sends the animal type indication information to the control apparatus 40. The control apparatus 40 determines the animal type carried by the animal type indication information as the animal type of the target animal. In another implementation, the control apparatus 40 receives the animal type of the target animal sent by another device such as a mobile phone or other terminals. In another implementation, the animal type of the blood sample is identified based on characteristic detection of the blood sample. In another implementation, an identification code such as a QR code is attached to a container such as a test tube of the blood sample, and an animal type recorded in the identification code is identified as the animal type of the target animal. Certainly, the control apparatus 40 may also determine the animal type of the target animal in other feasible ways.

The control apparatus 40 may determine the first target reticulocyte incubation time corresponding to the target animal based on the animal type of the target animal, and there may be included several specific implementations as follows. It can be understood that the first incubation time determined by the control apparatus 40 is related to reticulocyte fragmentation characteristics of the target animal, which may ensure that a fragmentation status of reticulocytes in the sample fluid to be tested that is prepared from the target animal within the first incubation time is within a preset range, thereby ensuring the accuracy of the reticulocyte testing result.

In one implementation, after determining the animal type of the target animal, the control apparatus 40 determines a reticulocyte incubation time corresponding to the animal type of the target animal from a plurality of preset correspondences between reticulocyte incubation times and animal types, and this incubation time is the first target reticulocyte incubation time. For example, three correspondences are preset, namely a reticulocyte incubation time corresponding to dogs (dog-25 seconds), a reticulocyte incubation time corresponding to cats (cats-15 seconds), and a reticulocyte incubation time corresponding to mice (mice-5 seconds). If the animal type of the target animal is determined to be a dog, the first target reticulocyte incubation time can be determined to be 25 seconds. It can be understood that for animals whose reticulocytes are relatively susceptible to fragmentation, corresponding reticulocyte incubation times are shorter; for animals whose reticulocytes are relatively less susceptible to fragmentation, corresponding reticulocyte incubation times are longer.

In this implementation, the preset correspondences between reticulocyte incubation times and animal types may be stored in the sample analyzer, or may be obtained by the sample analyzer from other devices. The sample analyzer stores a plurality of preset correspondences between reticulocyte incubation times and animal types, which may be stored based on custom information of the user. Specifically, the human-computer interaction apparatus 50 receives the custom information input by the user, and the custom information carries reticulocyte incubation times and animal types. The control apparatus 40 determines the reticulocyte incubation times and the animal types carried in the custom information as the preset correspondences between reticulocyte incubation times and animal types, and may further store the preset correspondences locally to the sample analyzer.

In another implementation, if the user finds that optional animal types provided by the sample analyzer do not include the animal type of the target animal, the user may input the animal type of the target animal and the corresponding first incubation time by adding a new animal type. Specifically, the human-computer interaction apparatus 50 receives the newly added animal information input by the user, the newly added animal information carrying the animal type and the reticulocyte incubation time corresponding to the animal type, and the control apparatus 40 determines the animal type carried by the newly added animal information as the animal type of the target animal, and determines the reticulocyte incubation time carried by the newly added animal information as the first incubation time corresponding to the target animal.

After determining the first incubation time corresponding to the target animal, the control apparatus 40 controls the incubation time of the sample fluid to be tested in the sample preparation apparatus 20 based on the first incubation time. The incubation time includes the reaction duration and/or the transfer duration of the sample fluid to be tested. Because the temperature of the sample fluid to be tested may affect the accuracy of reticulocyte testing results, the reaction cell is usually a constant temperature environment. The temperature of a pipeline is not easy to be controlled at a constant temperature. The sample fluid to be tested is usually transferred to the testing apparatus relatively quickly for testing. Therefore, in a specific implementation, the reaction duration of the sample fluid to be tested may be mainly controlled, while the transfer duration of the sample fluid to be tested may be ignored. A specific control method may be setting a timer for the reaction cell, and if the reaction duration of the sample fluid to be tested in the reaction cell reaches the first incubation time, controlling the transfer portion to transfer the sample fluid to be tested to the testing apparatus 30.

The control apparatus 40 includes at least a processing component with a data computing capability such as a processor, and a computer-readable storage medium. The computer-readable storage medium stores a computer program and may store data related to the computer program. When the computer program is called by the processor, the animal type of the target animal is determined, and the first target reticulocyte incubation time corresponding to the target animal is determined based on the animal type of the target animal. The incubation time of the sample fluid to be tested in the sample preparation apparatus is controlled based on the first target reticulocyte incubation time, and the testing result of the reticulocytes in the sample fluid to be tested is obtained based on the optical pulse signal of the cells in the sample fluid to be tested.

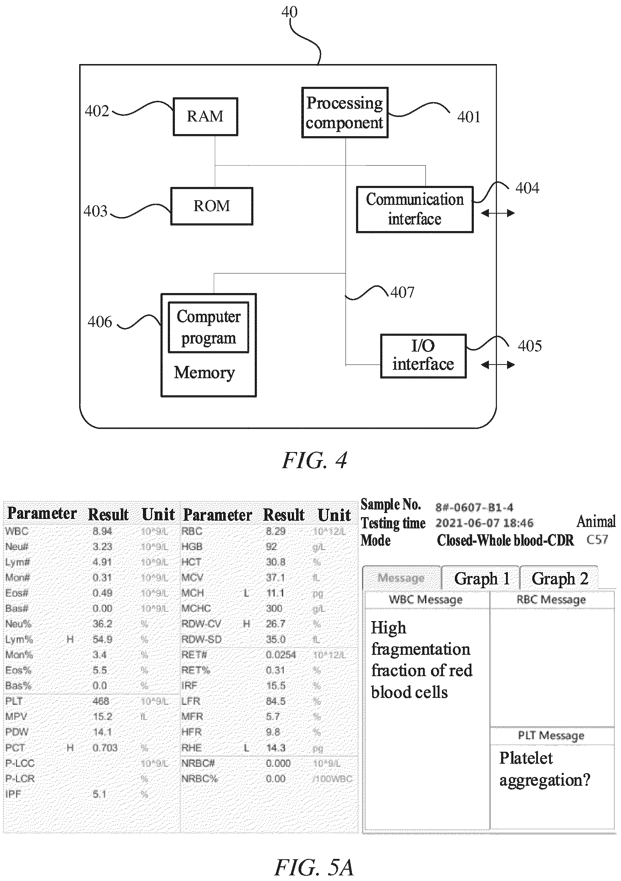

A specific structure of the control apparatus 40 is shown in FIG. 4. The control apparatus 40 includes at least a processing component 401, a random access memory (RAM) 402, a read-only memory (ROM) 403, a communication interface 404, an I/O interface 405, and a memory 406. The processing component 401, the RAM 402, the ROM 403, the communication interface 404, the I/O interface 405, and the memory 406 communicate via a bus 407. The processing component may be a central processing unit (CPU), a graphics processing unit (GPU), or other chips with computing capabilities. The memory 406 contains various computer programs for execution by the processor component 401 and data required for executing the computer programs. In addition, during the analysis process of the blood sample, if there is data that needs to be stored locally, such as the reticulocyte incubation time, the data may be stored in the memory 406. The I/O interface 405 is composed of a serial interface such as USB, IEEE 1394, or RS-232C, a parallel interface such as SCSI, IDE, or IEEE 1284, and an analog signal interface composed of a D/A converter, an A/D converter, etc. The I/O interface 405 may be connected to a display apparatus with a display function, such as a liquid crystal display, a touch screen, an LED display screen, etc. The control apparatus 40 may output the obtained data, such as the animal type, the first target reticulocyte incubation time, the testing result, the reticulocyte fragmentation status, etc., to the display apparatus for display. The communication interface 404 may be an interface of any currently known communication protocol, and the communication interface 404 communicates with the outside over a network. The control apparatus 40 may transmit, through the communication interface 404 using a specific communication protocol, data with any apparatus connected over the network. For example, the control apparatus 40 communicates with a computer device through the communication interface 404, and the computer device sends the plurality of preset correspondences between reticulocyte incubation times and animal types to the control apparatus 40 for storage. Alternatively, the control apparatus 40 is connected to a display through the communication interface 404, and sends the processed data such as the animal type, the first target reticulocyte incubation time, the testing result, the reticulocyte fragmentation status, etc. to the display for output and display. For another example, the control apparatus 40 communicates with a printer through the communication interface 404, and sends the processed data such as the animal type, the first target reticulocyte incubation time, the testing result, the reticulocyte fragmentation status, etc. to the printer for printing and output.

Based on the above description, it can be learned that compared with existing sample analyzers, after collecting the blood sample of the animal, the sample analyzer provided in this embodiment of the disclosure may determine the reticulocyte incubation time corresponding to the animal based on the animal type of the animal, and control an incubation time of the blood sample based on the reticulocyte incubation time, to prepare the sample fluid to be tested. Because a fragmentation status of reticulocytes in the sample fluid to be tested that is obtained based on this reticulocyte incubation time is within a preset reasonable range, the accuracy of a testing result of the reticulocytes is ensured. It can be learned that this sample analyzer may improve the accuracy of reticulocyte testing results of various different types of animals and expand the scope of application of animal testing.

In practical applications, in some cases, fragmentation characteristics of reticulocytes in blood samples of the same type of animals may be different. For example, after rats have undergone radioactive experiments or developed certain diseases, reticulocytes in the blood samples are more susceptible to fragmentation than those of rats that have not undergone radioactive experiments. Therefore, even if the sample fluid to be tested is subjected to incubation control for a reticulocyte incubation time corresponding to the animal type, a reticulocyte testing result may still not be accurate enough. In consideration of the above case, the sample analyzer provided in this embodiment of the disclosure may also perform more refined control over the incubation time, that is, on the basis of setting corresponding reticulocyte incubation times for different animal types, further sets a plurality of reticulocyte incubation times corresponding to the same animal type, different reticulocyte incubation times corresponding to reticulocyte fragmentation characteristics of the same type of animal under different circumstances.

To meet the above requirements, after obtaining the reticulocyte testing result, the control apparatus 40 of the sample analyzer may further determine, based on the testing result, a fragmentation status of the reticulocytes in the sample fluid to be tested, and if the fragmentation status meets a preset condition, control the human-computer interaction apparatus 50 to output prompt information for indicating an abnormal fragmentation amount of the reticulocytes. Specifically, the control apparatus 40 may determine the fragmentation status of the reticulocytes based on the reticulocyte testing result. The fragmentation status may be specifically a fragmentation amount or a fragmentation fraction. Once it is found that the fragmentation status of the reticulocytes meets the preset condition, it indicates that the fragmentation amount of the reticulocytes in the sample fluid to be tested is abnormal, and then the human-computer interaction apparatus 50 is controlled to output the prompt information. The abnormal fragmentation amount may be specifically a relatively high fragmentation fraction.

In a specific implementation, the testing apparatus of the sample analyzer may further include an impedance testing apparatus in addition to the optical testing apparatus. The control apparatus 40 may obtain a reticulocyte channel red blood cell count (RBC-O) from the reticulocyte testing result obtained by the optical testing apparatus, and obtain an impedance channel red blood cell count (RBC-I) measured by the impedance testing apparatus, and determine a ratio of the reticulocyte channel red blood cell count to the impedance channel red blood cell count (RBC-O/RBC-I) as the fragmentation fraction. If the fragmentation fraction is lower than a preset threshold, e.g., 0.93, it indicates that the reticulocyte fragmentation status meets the preset condition, and therefore the human-computer interaction apparatus 50 is controlled to output prompt information to prompt the user that the fragmentation fraction of the reticulocytes is relatively high. In one prompting method, the human-computer interaction apparatus 50 may display a testing result interface and include the prompt information in the testing result interface for prompting. FIG. 5A shows an example of a testing result interface, which shows prompt information "High fragmentation fraction of red blood cells" in a red blood cell information (WBC Message) region. In other prompting methods, the prompt information may be displayed independently of the reticulocyte testing result, or may be prompted in other manners such as sound, light, image, etc. It should be noted that the preset threshold described above is merely an example for description. The threshold may be any value from 0.90 to 0.95, or may be set to another value according to testing requirements in actual applications. This is not specifically limited in the disclosure.

An abnormal reticulocyte fragmentation status indicates that the blood sample of the target animal needs to be retested. This embodiment of the disclosure provides three retesting schemes.

In a first retesting scheme, the sample analyzer may prompt another reticulocyte incubation time for retesting. The first incubation time determined by the sample analyzer for the target animal based on the animal type by default is an incubation time required for the reticulocytes of the target animal under normal conditions. If the target animal undergoes a radioactive experiment or develops a disease, the abnormality mentioned above may occur because the fragmentation characteristics of the reticulocytes are changed (specifically, the reticulocytes become more susceptible to fragmentation). Therefore, when it is determined that the fragmentation status of the reticulocytes in the sample fluid to be tested meets the preset condition, the control apparatus 40 determines a second target reticulocyte incubation time (referred to as a second incubation time for short) that is shorter than the first target reticulocyte incubation time. Specifically, the control apparatus 40 may determine the second incubation time in a plurality of manners, such as by having the user input the second incubation time, or by pre-storing a plurality of incubation times for the same type of animal, and selecting another incubation time that is shorter than the first incubation time from the plurality of incubation times as the second incubation time, etc.

Further, when or after controlling the human-computer interaction apparatus 50 to output the prompt information of the abnormal fragmentation amount of the reticulocytes, the control apparatus 40 controls the human-computer interaction apparatus 50 to display the second target reticulocyte incubation time to prompt the user to retest the blood sample of the target animal based on the reset incubation time.

After controlling the sample analyzer to recollect a blood sample, the user may input a confirmation instruction to the sample analyzer, such that the sample analyzer retests the blood sample of the target animal based on the determined second incubation time. Specifically, the control apparatus 40 controls, in response to the confirmation instruction of the user for the second target reticulocyte incubation time, the incubation time of the sample fluid to be tested in the sample preparation apparatus based on the second target reticulocyte incubation time, to obtain another testing result of the reticulocytes in the sample fluid to be tested. It should be noted that the control process, based on the second incubation time, of the control apparatus 40 for the sample fluid to be tested is the same as the control process of the first incubation time above. For a specific implementation, reference may be made to the related description above, which is not repeated here.

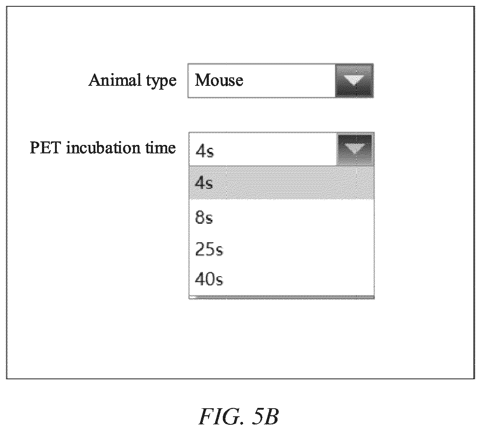

In a second retesting scheme, the human-computer interaction apparatus 50 may display a testing result interface, which includes the prompt information of the abnormal fragmentation amount of the reticulocytes and a jump control, and the jump control is linked to an incubation time adjustment interface. For example, the testing result interface may include a "Retest" control (which is a jump control). If the user determines, based on the prompt information, that the blood sample needs to be retested, the user may trigger the jump control. The control apparatus 40 controls, in response to a touch instruction of the user for the jump control, the human-computer interaction apparatus to display the incubation time adjustment interface, the incubation time adjustment interface including a plurality of reticulocyte incubation times corresponding to the animal type of the target animal, wherein at least one of the plurality of reticulocyte incubation times is shorter than the first target reticulocyte incubation time. FIG. 5B shows an example of the incubation time adjustment interface. Assuming that the target animal is a mouse, the user triggers the "Retest" control on the testing result interface and jumps to the incubation time adjustment interface shown in FIG. 5B, which includes an animal type control and a RET incubation time control. The animal type control may automatically display the type of the target animal, i.e., "Mouse". After the user triggers the RET incubation time control, a plurality of reticulocyte incubation times (4s, 8s, 25s, and 40s) corresponding to the animal type "Mouse" may be displayed. The user may select one of the reticulocyte incubation times as the second target reticulocyte incubation time. It should be noted that to help the user select a suitable reticulocyte incubation time (i.e., a reticulocyte incubation time that is shorter than the first target reticulocyte incubation time), the first target reticulocyte incubation time before retesting may be prompted on this interface. For example, when the incubation time adjustment interface is displayed, a time displayed by the RET incubation time control may be set to 40s as the first target reticulocyte incubation time. According to this prompt, the user may select any reticulocyte incubation time from 4s, 8s, and 25s for retesting.

According to the prompt on the incubation time adjustment interface, the user may select another reticulocyte incubation time shorter than the first incubation time from the plurality of reticulocyte incubation times as the second incubation time. In order to help the user select a suitable second incubation time, the first incubation time may be distinguished from other reticulocyte incubation times on the incubation time adjustment interface. The control apparatus 40 controls, in response to an instruction of the user to select the second target reticulocyte incubation time, the incubation time of the sample fluid to be tested in the sample preparation apparatus based on the second target reticulocyte incubation time, to obtain another testing result of the reticulocytes in the sample fluid to be tested, wherein the second target reticulocyte incubation time is shorter than the first target reticulocyte incubation time. It should be noted that the control process, based on the second incubation time, of the control apparatus 40 for the sample fluid to be tested is the same as the control process of the first incubation time above. For a specific implementation, reference may be made to the related description above, which is not repeated here.

Compared with the first retesting scheme, by using this retesting scheme, the user may quickly jump from the testing result interface to the incubation time adjustment interface to adjust the incubation time, making retesting more convenient and quicker. Certainly, the testing result interface may not include the jump control, and the user may manually switch to a home page or another retesting interface to manually trigger the retesting process.

In a third retesting scheme, the sample analyzer may automatically perform retesting. Specifically, if the reticulocyte fragmentation status meets the preset condition, the control apparatus 40 determines a second target reticulocyte incubation time that is shorter than the first target reticulocyte incubation time, and controls the incubation time of the sample fluid to be tested in the sample preparation apparatus based on the second target reticulocyte incubation time, to obtain another testing result of the reticulocytes in the sample fluid to be tested. It should be noted that the control process, based on the second incubation time, of the control apparatus 40 for the sample fluid to be tested is the same as the control process of the first incubation time above. For a specific implementation, reference may be made to the related description above, which is not repeated here.

Compared with the first two retesting schemes, by using this retesting scheme, the user only needs to re-prepare a blood sample of the target animal according to the prompt information, and the sample analyzer may automatically adjust the incubation time and retest the blood sample based on the adjusted incubation time, making the retesting more automatic and quicker.

In practical applications, abnormal reticulocyte testing results may be caused by abnormal fragmentation amounts of reticulocytes, or may be caused by abnormal staining statuses of reticulocytes in the sample fluid to be tested. Based on the reticulocyte testing processes shown in FIG. 3A and FIG. 3B, it can be learned that a staining solution needs to be added to the blood sample, and the staining solution is used to stain the reticulocytes. Only the stained reticulocytes can be tested by the testing apparatus. If the reticulocyte staining status is abnormal, the accuracy of reticulocyte testing results may also be affected.

For example, referring to the experimental data in Table 1 and Table 4, the incubation times of the six dog blood samples in Table 4 are 8 seconds, and the incubation times of the six dog blood samples in Table 1 are 25 seconds. The accuracy of the reticulocyte testing results in Table 4 is lower than that in Table 1, reasons for which, as inferred, may be: the incubation times in Table 4 are short, and therefore the staining time of reticulocytes in the dog blood sample is short, thus the accuracy of the reticulocyte testing results is affected due to insufficient staining of the reticulocytes.

Therefore, the sample analyzer provided in this embodiment of the disclosure may further set corresponding incubation times for different types of animals based on reticulocyte staining characteristics of the animals, so as to ensure the accuracy of reticulocyte testing results of different types of animals.

Similar to the retesting scheme for abnormal reticulocyte fragmentation, this embodiment of the disclosure provides three retesting schemes for abnormal staining.

In a first retesting scheme, the control apparatus 40 determines, based on the testing result, a staining status of the reticulocytes in the sample fluid to be tested; if the staining status meets a preset condition, the human-computer interaction apparatus is controlled to output prompt information for indicating an abnormal staining amount of the reticulocytes. The sample analyzer may automatically detect the staining status of the reticulocytes, such as a staining percentage; and when it is found that the staining percentage is abnormal, specifically, the staining percentage is relatively low, prompt information is output by the human-computer interaction apparatus 50. For the prompt information, reference may be made to the related description above of the fragmentation status, which is not repeated here.

In another specific implementation, if the user finds that the reticulocyte staining is abnormal, the user may input a staining abnormality instruction to the sample analyzer through the human-computer interaction apparatus 50. For example, the testing result interface of the human-computer interaction apparatus 50 may include an "Abnormal staining" control or the sample analyzer may be provided with a physical button corresponding to staining abnormality, and the user inputs the staining abnormality instruction by triggering the control or the physical button. The control apparatus 40 determines, in response to the staining abnormality instruction input by the user, a third target reticulocyte incubation time that is longer than the first target reticulocyte incubation time, and controls the human-computer interaction apparatus to display the third target reticulocyte incubation time (referred to as a third incubation time). For a method for determining the third target reticulocyte incubation time, reference may be made to the method for determining the second target reticulocyte incubation time, which is not repeated here.

If the user confirms that the third incubation time prompted by the human-computer interaction apparatus 50 is proper, the user may input a confirmation instruction based on the human-computer interaction apparatus 50, and the control apparatus 40 controls, in response to the confirmation instruction of the user for the third target reticulocyte incubation time, the incubation time of the sample fluid to be tested in the sample preparation apparatus based on the third target reticulocyte incubation time, to obtain another testing result of the reticulocytes in the sample fluid to be tested. It should be noted that the third incubation time is longer than the first incubation time, which may ensure that the reticulocytes in the sample fluid to be tested are fully stained, thereby improving the accuracy of the reticulocyte testing result.

In a second retesting scheme, the control apparatus 40 displays an incubation time adjustment interface in response to a staining abnormality instruction input by the user based on the human-computer interaction apparatus. The incubation time adjustment interface includes a plurality of reticulocyte incubation times corresponding to the animal type of the target animal, and at least one of the plurality of reticulocyte incubation times is longer than the first target reticulocyte incubation time. The control apparatus 40 controls, in response to an instruction of the user to select a third target reticulocyte incubation time from the plurality of reticulocyte incubation times, the incubation time of the sample fluid to be tested in the sample preparation apparatus based on the third target reticulocyte incubation time, to obtain another testing result of the reticulocytes in the sample fluid to be tested.

The retesting scheme may prompt the user of a plurality of reticulocyte incubation times, from which the user may select the third incubation time that is longer than the first incubation time.

**In** a third retesting scheme, the sample analyzer automatically performs retesting based on a staining abnormality instruction of the user. Specifically, the control apparatus 40 determines, in response to the staining abnormality instruction input by the user based on the human-computer interaction apparatus, a third target reticulocyte incubation time that is longer than the first target reticulocyte incubation time, and controls the incubation time of the sample fluid to be tested in the sample preparation apparatus based on the third target reticulocyte incubation time, to obtain another testing result of the reticulocytes in the sample fluid to be tested.

The control process, based on the second incubation time, of the control apparatus 40 for the sample fluid to be tested is the same as the control process of the first incubation time above. For a specific implementation, reference may be made to the related description above, which is not repeated here.

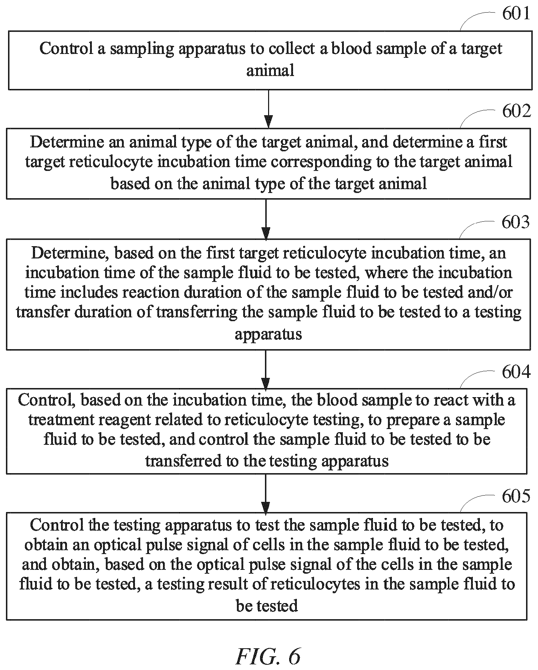

Corresponding to the sample analyzer, an embodiment of the disclosure further provides an animal reticulocyte testing method, which may be specifically implemented by a control apparatus. As shown in FIG. 6, the animal reticulocyte testing method may specifically include steps 601 to 605.

601: Control a sampling apparatus to collect a blood sample of a target animal.

602: Determine an animal type of the target animal, and determine a first target reticulocyte incubation time corresponding to the target animal based on the animal type of the target animal.

603: Determine, based on the first target reticulocyte incubation time, an incubation time of the sample fluid to be tested, wherein the incubation time includes reaction duration of the sample fluid to be tested and/or transfer duration of transferring the sample fluid to be tested to a testing apparatus.

604: Control, based on the incubation time, the blood sample to react with a treatment reagent related to reticulocyte testing, to prepare a sample fluid to be tested, and control the sample fluid to be tested to be transferred to the testing apparatus.

605: Control the testing apparatus to test the sample fluid to be tested, to obtain an optical pulse signal of cells in the sample fluid to be tested, and obtain, based on the optical pulse signal of the cells in the sample fluid to be tested, a testing result of reticulocytes in the sample fluid to be tested.

In one specific implementation, determining an animal type of the target animal includes: receiving animal type indication information, wherein the animal type indication information carries an animal type; and determining the animal type carried by the animal type indication information as the animal type of the target animal.

In one specific implementation, determining a first target reticulocyte incubation time corresponding to the target animal based on the animal type of the target animal includes:
determining a reticulocyte incubation time corresponding to the animal type of the target animal from a plurality of preset correspondences between reticulocyte incubation times and animal types, and using the determined reticulocyte incubation time as the first target reticulocyte incubation time corresponding to the target animal.

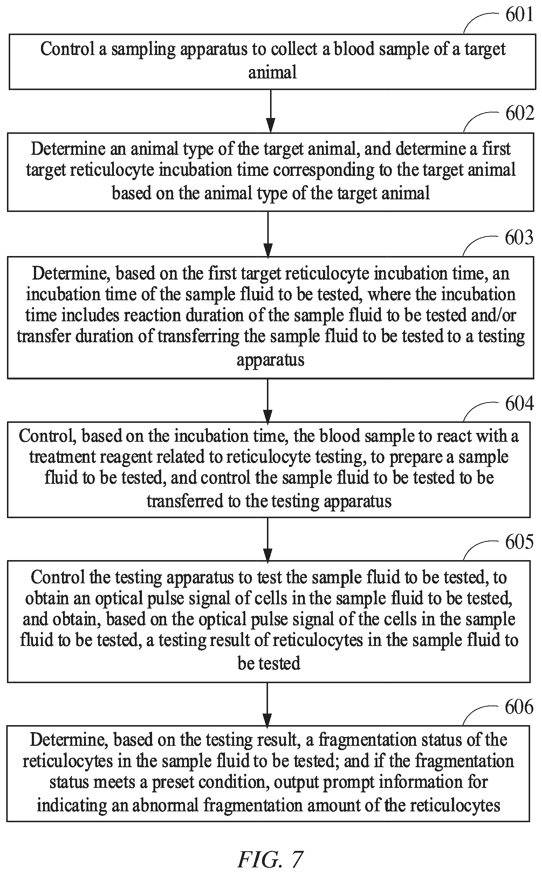

As shown in FIG. 7, the animal reticulocyte testing method provided in this embodiment of the disclosure further includes step 606 on the basis of the process shown in FIG. 6: determining, based on the testing result, a fragmentation status of the reticulocytes in the sample fluid to be tested; and if the fragmentation status meets a preset condition, outputting prompt information for indicating an abnormal fragmentation amount of the reticulocytes.

In one specific implementation, the animal reticulocyte testing method further includes: determining a second target reticulocyte incubation time that is shorter than the first target reticulocyte incubation time; and displaying the second target reticulocyte incubation time when or after outputting the prompt information indicating the abnormal fragmentation amount of the reticulocytes.

In one specific implementation, the animal reticulocyte testing method further includes: controlling, based on the second target reticulocyte incubation time in response to a confirmation instruction of a user for the second target reticulocyte incubation time, the incubation time of the sample fluid to be tested, to obtain another testing result of the reticulocytes in the sample fluid to be tested.

In one specific implementation, outputting prompt information for indicating an abnormal fragmentation amount of the reticulocytes includes: displaying a testing result interface, wherein the testing result interface includes the prompt information of the abnormal fragmentation amount of the reticulocytes and a jump control.

The method further includes: displaying an incubation time adjustment interface in response to a touch instruction of the user for the jump control, the incubation time adjustment interface including a plurality of reticulocyte incubation times corresponding to the animal type of the target animal, wherein at least one of the plurality of reticulocyte incubation times is shorter than the first target reticulocyte incubation time.

In one specific implementation, the animal reticulocyte testing method further includes: controlling, based on the second target reticulocyte incubation time in response to an instruction of the user to select a second target reticulocyte incubation time from the plurality of reticulocyte incubation times, the incubation time of the sample fluid to be tested, to obtain another testing result of the reticulocytes in the sample fluid to be tested, wherein the second target reticulocyte incubation time is shorter than the first target reticulocyte incubation time.

In one specific implementation, the animal reticulocyte testing method further includes: if the staining status meets a preset condition, determining a third target reticulocyte incubation time that is longer than the first target reticulocyte incubation time, and controlling, based on the third target reticulocyte incubation time, the incubation time of the sample fluid to be tested, to obtain another testing result of the reticulocytes in the sample fluid to be tested.

In one specific implementation, the animal reticulocyte testing method further includes: determining, in response to a staining abnormality instruction input by the user, a third target reticulocyte incubation time that is longer than the first target reticulocyte incubation time, and displaying the third target reticulocyte incubation time.

In one specific implementation, the animal reticulocyte testing method further includes: controlling, based on the third target reticulocyte incubation time in response to a confirmation instruction of the user for the third target reticulocyte incubation time, the incubation time of the sample fluid to be tested, to obtain another testing result of the reticulocytes in the sample fluid to be tested.

In one specific implementation, the animal reticulocyte testing method further includes displaying an incubation time adjustment interface in response to a staining abnormality instruction input by the user, wherein the incubation time adjustment interface includes a plurality of reticulocyte incubation times corresponding to the animal type of the target animal, and at least one of the plurality of reticulocyte incubation times is longer than the first target reticulocyte incubation time.

In one specific implementation, the animal reticulocyte testing method further includes controlling, based on the third target reticulocyte incubation time in response to an instruction of the user to select a third target reticulocyte incubation time from the plurality of reticulocyte incubation times, the incubation time of the sample fluid to be tested, to obtain another testing result of the reticulocytes in the sample fluid to be tested, wherein the third target reticulocyte incubation time is longer than the first target reticulocyte incubation time.

In one specific implementation, the animal reticulocyte testing method further includes determining, in response to a staining abnormality instruction input by the user, a third target reticulocyte incubation time that is longer than the first target reticulocyte incubation time, and controlling, based on the third target reticulocyte incubation time, the incubation time of the sample fluid to be tested, to obtain another testing result of the reticulocytes in the sample fluid to be tested.

In one specific implementation, the animal reticulocyte testing method further includes determining, based on the testing result, a staining status of the reticulocytes in the sample fluid to be tested; and if the staining status meets a preset condition, outputting prompt information for indicating an abnormal staining amount of the reticulocytes.

With respect to the above description of the disclosed embodiments, the features recorded in the embodiments in this description may be replaced or combined with each other, so that those skilled in the art could implement or use the disclosure. Descriptions are provided herein with reference to various exemplary embodiments. However, those skilled in the art should understand that changes and corrections may be made to the exemplary embodiments without departing from the scope of the appended claims. For example, various operational steps and assemblies for executing the operational steps may be implemented in different methods on the basis of specific applications or in consideration of any number of cost functions associated with operations of the system (for example, one or more steps may be deleted, modified or incorporated into other steps).

The terms "first", "second", etc. in the specification and the claims herein as well as the above accompanying drawings are used to distinguish different objects, rather than to describe a specific order. In addition, the terms "comprise", "have", and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these processes, methods, or devices.

In addition, those skilled in the art can understand that the principles herein may be reflected in a computer program product in a computer-readable storage medium, wherein the readable storage medium is loaded with computer-readable program codes in advance. Any tangible non-transitory computer-readable storage medium may be used, including a magnetic storage device (a hard disk, a floppy disk, or the like), an optical storage device (CD-ROM, DVD, Blu-ray disc, or the like), a flash memory, and/or the like. These computer program instructions may be loaded on a general-purpose computer, a special-purpose computer, or other programmable data processing devices to form a machine, such that these instructions, when executed on a computer or other programmable data processing apparatuses, may produce a means for implementing a specified function. These computer program instructions may alternatively be stored in a computer-readable memory. The computer-readable memory may instruct a computer or other programmable data processing devices to operate in a particular manner, such that the instructions stored in the computer-readable memory may produce an article of manufacture which includes a means for implementing a specified function. The computer program instructions may alternatively be loaded onto a computer or other programmable data processing devices to perform a series of operational steps on the computer or other programmable devices to produce a computer-implemented process, such that the instructions, when executed on the computer or other programmable devices, may provide steps for implementing a specified function.

The foregoing detailed descriptions are provided with reference to various embodiments. However, those skilled in the art should understand that various corrections and changes may be made without departing from the scope of the appended claims. Therefore, the disclosure is intended for an illustrative purpose other than a limitative purpose, and all these modifications fall within the scope of the disclosure. Similarly, the advantages, other advantages, and solutions to problems of the various embodiments are described above. However, the benefits, the advantages, the solutions to the problems, and any of their contributing factors, or solutions clarifying them should not be construed to be critical, necessary, or essential. The term "include" used herein and any other variations thereof all refer to a non-exclusive inclusion, such that a process, method, article, or device including a list of elements includes not only these elements, but also other elements that are not expressly listed or not inherent to the process, method, system, article, or device. In addition, the term "couple" used herein and any other variations thereof refer to a physical connection, an electrical connection, a magnetic connection, an optical connection, a communication connection, a functional connection, and/or any other connections.

The above embodiments merely represent several embodiments, giving specifics and details thereof, but should not be understood as limiting the scope of the appended claims. It should be noted that those of ordinary skill in the art would have also made several variations and improvements without departing from the concept of the disclosure, and these variations and improvements would all fall within the scope of protection of the disclosure. Therefore, the scope of protection of the disclosure shall be in accordance with the appended claims.

## Claims

1. A sample analyzer, wherein the sample analyzer comprises:
a sampling apparatus (10) configured to collect a blood sample of a target animal and transfer the blood sample to a sample preparation apparatus (20);
the sample preparation apparatus (20) having a reaction cell and a transfer portion, wherein the reaction cell is configured to provide a reaction site for the blood sample to react with a treatment reagent related to reticulocyte testing, to prepare a sample fluid to be tested, and the transfer portion is configured to transfer the sample fluid to be tested in the reaction cell to a testing apparatus (30), wherein the treatment reagent comprises a reticulocyte diluent, and an osmotic pressure of the reticulocyte diluent is lower than an osmotic pressure of reticulocytes in the sample fluid to be tested;
the testing apparatus (30) configured to test the sample fluid to be tested, to obtain an optical pulse signal of cells in the sample fluid to be tested; and
a control apparatus (40) communicatively connected to the sample preparation apparatus (20) and the testing apparatus (30), and configured to: determine an animal type of the target animal, and determine a first target reticulocyte incubation time corresponding to the target animal based on the animal type of the target animal, so as to reduce a fragmentation of the reticulocytes in the sample fluid to be tested; control an incubation time of the sample fluid to be tested in the sample preparation apparatus (20) based on the first target reticulocyte incubation time, wherein the incubation time comprises reaction duration of the sample fluid to be tested in the reaction cell and/or transfer duration of transferring the sample fluid to be tested to the testing apparatus (30) by the transfer portion; and obtain, based on the optical pulse signal of the cells in the sample fluid to be tested, a testing result of the reticulocytes in the sample fluid to be tested.

2. The sample analyzer of claim 1, **characterized in that** the sample analyzer further comprises a human-computer interaction apparatus (50),
wherein the human-computer interaction apparatus (50) is communicatively connected to the control apparatus (40), and is configured to receive animal type indication information, the animal type indication information carrying the animal type; and send the animal type indication information to the control apparatus (40); and
when performing the step of determining an animal type of the target animal, the control apparatus (40) is specifically configured to determine the animal type carried by the animal type indication information as the animal type of the target animal.

3. The sample analyzer of claim 1, **characterized in that** when performing the step of determining a first target reticulocyte incubation time corresponding to the target animal based on the animal type of the target animal, the control apparatus (40) is specifically configured to:
determine a reticulocyte incubation time corresponding to the animal type of the target animal from a plurality of preset correspondences between reticulocyte incubation times and animal types, and use the determined reticulocyte incubation time as the first target reticulocyte incubation time corresponding to the target animal.

4. The sample analyzer of claim 3, **characterized in that** the sample analyzer further comprises a human-computer interaction apparatus (50), wherein
the human-computer interaction apparatus (50) is communicatively connected to the control apparatus (40), and is configured to receive custom information, the custom information carrying reticulocyte incubation times and animal types; and send the custom information to the control apparatus (40); and
the control apparatus (40) is further configured to determine the reticulocyte incubation times and the animal types carried by the custom information as the preset correspondences between reticulocyte incubation times and animal types.

5. The sample analyzer of claim 1, **characterized in that** the sample analyzer further comprises a human-computer interaction apparatus (50) communicatively connected to the control apparatus (40), wherein the control apparatus (40) is further configured to:
determine, based on the testing result, a fragmentation status of the reticulocytes in the sample fluid to be tested; and
if the fragmentation status meets a preset condition, control the human-computer interaction apparatus (50) to output prompt information for indicating an abnormal fragmentation amount of the reticulocytes.

6. The sample analyzer of claim 5, **characterized in that** the control apparatus (40) is further configured to:
determine a second target reticulocyte incubation time that is shorter than the first target reticulocyte incubation time, and control the human-computer interaction apparatus (50) to display the second target reticulocyte incubation time when or after controlling the human-computer interaction apparatus (50) to output the prompt information indicating the abnormal fragmentation amount of the reticulocytes.

7. The sample analyzer of claim 5, **characterized in that**
the human-computer interaction apparatus (50) is specifically configured to display a testing result interface, wherein the testing result interface comprises the prompt information for the abnormal fragmentation amount of the reticulocytes and a jump control; and
the control apparatus (40) is further configured to control, in response to a touch instruction of the user for the jump control, the human-computer interaction apparatus (50) to display an incubation time adjustment interface, the incubation time adjustment interface comprising a plurality of reticulocyte incubation times corresponding to the animal type of the target animal, wherein at least one of the plurality of reticulocyte incubation times is shorter than the first target reticulocyte incubation time.

8. The sample analyzer of claim 7, **characterized in that** the control apparatus (40) is further configured to:
control, in response to an instruction of the user to select a second target reticulocyte incubation time from the plurality of reticulocyte incubation times, the incubation time of the sample fluid to be tested in the sample preparation apparatus (20) based on the second target reticulocyte incubation time, to obtain another testing result of the reticulocytes in the sample fluid to be tested, wherein the second target reticulocyte incubation time is shorter than the first target reticulocyte incubation time.

9. The sample analyzer of claim 1, **characterized in that** the sample analyzer comprises a human-computer interaction apparatus (50) communicatively connected to the control apparatus (40), wherein
the control apparatus (40) is further configured to determine, in response to a staining abnormality instruction input by the user based on the human-computer interaction apparatus (50), a third target reticulocyte incubation time that is longer than the first target reticulocyte incubation time, and control the human-computer interaction apparatus (50) to display the third target reticulocyte incubation time.

10. The sample analyzer of claim 9, **characterized in that**
the control apparatus (40) is further configured to control, in response to a confirmation instruction of the user for the third target reticulocyte incubation time, the incubation time of the sample fluid to be tested in the sample preparation apparatus (20) based on the third target reticulocyte incubation time, to obtain another testing result of the reticulocytes in the sample fluid to be tested.

11. The sample analyzer of claim 1, **characterized in that** the sample analyzer further comprises a human-computer interaction apparatus (50) communicatively connected to the control apparatus (40), wherein
the control apparatus (40) is further configured to display an incubation time adjustment interface in response to a staining abnormality instruction input by the user based on the human-computer interaction apparatus (50), wherein the incubation time adjustment interface comprises a plurality of reticulocyte incubation times corresponding to the animal type of the target animal, and at least one of the plurality of reticulocyte incubation times is longer than the first target reticulocyte incubation time.

12. The sample analyzer of claim 11, **characterized in that** the control apparatus (40) is further configured to:
control, in response to an instruction of the user to select a third target reticulocyte incubation time from the plurality of reticulocyte incubation times, wherein the third target reticulocyte incubation time is longer than the first target reticulocyte incubation time, and control the incubation time of the sample fluid to be tested in the sample preparation apparatus (20) based on the third target reticulocyte incubation time, to obtain another testing result of the reticulocytes in the sample fluid to be tested.

13. An animal reticulocyte testing method, wherein the method comprises:
controlling (601) a sampling apparatus to collect a blood sample of a target animal;
determining (602) an animal type of the target animal, and determining a first target reticulocyte incubation time corresponding to the target animal based on the animal type of the target animal, so as to reduce a fragmentation of reticulocytes in the sample fluid to be tested;
determining (603), based on the first target reticulocyte incubation time, an incubation time of the sample fluid to be tested, wherein the incubation time comprises reaction duration of the sample fluid to be tested and/or transfer duration of transferring the sample fluid to be tested to a testing apparatus; and
controlling (604), based on the incubation time, the blood sample to react with a treatment reagent related to reticulocyte testing, to prepare a sample fluid to be tested, and controlling the sample fluid to be tested to be transferred to the testing apparatus, wherein the treatment reagent comprises a reticulocyte diluent, and an osmotic pressure of the reticulocyte diluent is lower than an osmotic pressure of the reticulocytes in the sample fluid to be tested; and
controlling (605) the testing apparatus to test the sample fluid to be tested, to obtain an optical pulse signal of cells in the sample fluid to be tested, and obtaining, based on the optical pulse signal of the cells in the sample fluid to be tested, a testing result of the reticulocytes in the sample fluid to be tested.

14. The animal reticulocyte testing method of claim 13, **characterized in that** the method further comprises:
determining, based on the testing result, a fragmentation status of the reticulocytes in the sample fluid to be tested; and
if the fragmentation status meets a preset condition, outputting prompt information for indicating an abnormal fragmentation amount of the reticulocytes.

15. The animal reticulocyte testing method of claim 14, **characterized in that** outputting prompt information for indicating an abnormal fragmentation amount of the reticulocytes comprises: displaying a testing result interface, wherein the testing result interface comprises the prompt information of the abnormal fragmentation amount of the reticulocytes and a jump control; and
the method further comprises:
displaying an incubation time adjustment interface in response to a touch instruction of the user for the jump control, the incubation time adjustment interface comprising a plurality of reticulocyte incubation times corresponding to the animal type of the target animal, wherein at least one of the plurality of reticulocyte incubation times is shorter than the first target reticulocyte incubation time.

## Patentansprüche

1. Probenanalysegerät, wobei das Probenanalysegerät umfasst:
eine Probenahmevorrichtung (10), die ausgestaltet ist zum Gewinnen einer Blutprobe eines Zieltiers und zum Transferieren der Blutprobe in eine Probenvorbereitungsvorrichtung (20);
wobei die Probenvorbereitungsvorrichtung (20) eine Reaktionszelle und einen Transferabschnitt aufweist, wobei die Reaktionszelle ausgestaltet ist, um einen Reaktionsort bereitzustellen, an dem die Blutprobe mit einem Behandlungsreagenz, das mit Retikulozytentests in Beziehung steht, reagieren kann, um eine zu testende Probenflüssigkeit vorzubereiten, und der Transferabschnitt ausgestaltet ist, um die zu testende Probenflüssigkeit in der Reaktionszelle zu einer Testvorrichtung (30) zu transferieren, wobei das Behandlungsreagenz ein Retikulozytenverdünnungsmittel umfasst, und ein osmotischer Druck des Retikulozytenverdünnungsmittels niedriger als ein osmotischer Druck von Retikulozyten in der zu testenden Probenflüssigkeit ist;
die Testvorrichtung (30) ausgestaltet ist zum Testen der zu testenden Probenflüssigkeit, um ein optisches Pulssignal von Zellen in der zu testenden Probenflüssigkeit zu erhalten; und
eine Steuervorrichtung (40), die kommunikativ mit der Probenvorbereitungsvorrichtung (20) und der Testvorrichtung (30) verbunden ist, und ausgestaltet ist zum: Bestimmen eines Tiertyps des Zieltiers, und Bestimmen einer ersten Zielretikulozyteninkubationszeit entsprechend dem Zieltier basierend auf dem Tiertyp des Zieltiers, um so eine Fragmentierung der Retikulozyten in der zu testenden Probenflüssigkeit zu reduzieren; Steuern einer Inkubationszeit der zu testenden Probenflüssigkeit in der Probenvorbereitungsvorrichtung (20) basierend auf der ersten Zielretikulozyteninkubationszeit, wobei die Inkubationszeit Reaktionsdauer der zu testenden Probenflüssigkeit in der Reaktionszelle und/oder Transferdauer des Transferierens der zu testenden Probenflüssigkeit zu der Testvorrichtung (30) durch den Transferabschnitt umfasst; und Erhalten eines Testergebnisses der Retikulozyten in der zu testenden Probenflüssigkeit basierend auf dem optischen Pulssignal der Zellen in der zu testenden Probenflüssigkeit.

2. Probenanalysegerät nach Anspruch 1, **dadurch gekennzeichnet, dass** das Probenanalysegerät des Weiteren eine Mensch-Computer-Interaktionsvorrichtung (50) umfasst,
wobei die Mensch-Computer-Interaktionsvorrichtung (50) kommunikativ mit der Steuervorrichtung (40) verbunden ist und ausgestaltet ist zum Empfangen von Tiertypangabeinformationen, wobei die Tiertypangabeinformationen den Tiertyp tragen; und Senden der Tiertypangabeinformationen an die Steuervorrichtung (40); und
wobei die Steuervorrichtung (40) speziell ausgestaltet ist zum Bestimmen des Tiertyps, der durch die Tiertypangabeinformationen getragen wird, als den Tiertyp des Zieltiers, wenn der Schritt des Bestimmens eines Tiertyps des Zieltiers durchgeführt wird.

3. Probenanalysegerät nach Anspruch 1, **dadurch gekennzeichnet, dass** die Steuervorrichtung (40), wenn der Schritt des Bestimmens einer ersten Zielretikulozyteninkubationszeit entsprechend dem Zieltier basierend auf dem Tiertyp des Zieltiers durchgeführt wird, speziell ausgestaltet ist zum:
Bestimmen einer Retikulozyteninkubationszeit entsprechend dem Tiertyp des Zieltiers aus einer Vielzahl voreingestellter Entsprechungen zwischen Retikulozyteninkubationszeiten und Tiertypen, und Verwenden der bestimmten Retikulozyteninkubationszeit als die erste Zielretikulozyteninkubationszeit entsprechend dem Zieltier.

4. Probenanalysegerät nach Anspruch 3, **dadurch gekennzeichnet, dass** das Probenanalysegerät des Weiteren eine Mensch-Computer-Interaktionsvorrichtung (50) umfasst, wobei
die Mensch-Computer-Interaktionsvorrichtung (50) kommunikativ mit der Steuervorrichtung (40) verbunden ist und ausgestaltet ist zum Empfangen von benutzerspezifischen Informationen, wobei die benutzerspezifischen Informationen Retikulozyteninkubationszeiten und Tiertypen tragen; und Senden der benutzerspezifischen Informationen an die Steuervorrichtung (40); und
die Steuervorrichtung (40) des Weiteren ausgestaltet ist zum Bestimmen der Retikulozyteninkubationszeiten und der Tiertypen, die von den benutzerspezifischen Informationen getragen werden, als die voreingestellten Entsprechungen zwischen Retikulozyteninkubationszeiten und Tiertypen.

5. Probenanalysegerät nach Anspruch 1, **dadurch gekennzeichnet, dass** das Probenanalysegerät des Weiteren eine Mensch-Computer-Interaktionsvorrichtung (50) umfasst, die kommunikativ mit der Steuervorrichtung (40) verbunden ist, wobei die Steuervorrichtung (40) des Weiteren ausgestaltet ist zum:
Bestimmen eines Fragmentierungsstatus der Retikulozyten in der zu testenden Probenflüssigkeit basierend auf dem Testergebnis; und
falls der Fragmentierungsstatus eine voreingestellte Bedingung erfüllt, Steuern der Mensch-Computer-Interaktionsvorrichtung (50), um Meldungsinformationen auszugeben, um eine abnormale Fragmentierungsmenge der Retikulozyten anzugeben.

6. Probenanalysegerät nach Anspruch 5, **dadurch gekennzeichnet, dass** die Steuervorrichtung (40) des Weiteren ausgestaltet ist zum:
Bestimmen einer zweiten Zielretikulozyteninkubationszeit, die kürzer als die erste Zielretikulozyteninkubationszeit ist, und Steuern der Mensch-Computer-Interaktionsvorrichtung (50) zum Anzeigen der zweiten Zielretikulozyteninkubationszeit, wenn die Mensch-Computer-Interaktionsvorrichtung (50) zum Ausgeben der Meldungsinformation gesteuert wird, welche die abnormale Fragmentierungsmenge der Retikulozyten angibt, oder danach.

7. Probenanalysegerät nach Anspruch 5, **dadurch gekennzeichnet, dass**
die Mensch-Computer-Interaktionsvorrichtung (50) speziell ausgestaltet ist zum Anzeigen einer Testergebnisschnittstelle, wobei die Testergebnisschnittstelle die Meldungsinformationen für die abnormale Fragmentierungsmenge der Retikulozyten und eine Sprungsteuerung umfasst; und
die Steuervorrichtung (40) des Weiteren ausgestaltet ist zum Steuern der Mensch-Computer-Interaktionsvorrichtung (50) in Reaktion auf eine Berührungsanweisung des Benutzers auf der Sprungsteuerung, um eine Inkubationszeitanpassungsschnittstelle anzuzeigen, wobei die Inkubationszeitanpassungsschnittstelle eine Vielzahl von Retikulozyteninkubationszeiten entsprechend dem Tiertyp des Zieltiers umfasst, wobei mindestens eine von der Vielzahl der Retikulozyteninkubationszeiten kürzer als die erste Zielretikulozyteninkubationszeit ist.

8. Probenanalysegerät nach Anspruch 7, **dadurch gekennzeichnet, dass** die Steuervorrichtung (40) des Weiteren ausgestaltet ist zum:
Steuern der Inkubationszeit der zu testenden Probenflüssigkeit in der Probenvorbereitungsvorrichtung (20) in Reaktion auf eine Anweisung des Benutzers, eine zweite Zielretikulozyteninkubationszeit aus der Vielzahl der Retikulozyteninkubationszeiten auszuwählen, basierend auf der zweiten Zielretikulozyteninkubationszeit, um ein anderes Testergebnis der Retikulozyten in der zu testenden Probenflüssigkeit zu erhalten, wobei die zweite Zielretikulozyteninkubationszeit kürzer als die erste Zielretikulozyteninkubationszeit ist.

9. Probenanalysegerät nach Anspruch 1, **dadurch gekennzeichnet, dass** das Probenanalysegerät eine Mensch-Computer-Interaktionsvorrichtung (50) umfasst, die kommunikativ mit der Steuervorrichtung (40) verbunden ist, wobei die Steuervorrichtung (40) des Weiteren ausgestaltet ist zum Bestimmen einer dritten Zielretikulozyteninkubationszeit, die länger als die erste Zielretikulozyteninkubationszeit ist, in Reaktion auf eine Färbungsabnormalitätsanweisung, die durch den Benutzer basierend auf der Mensch-Computer-Interaktionsvorrichtung (50) eingegeben wurde, und zum Steuern der Mensch-Computer-Interaktionsvorrichtung (50) zum Anzeigen der dritten Zielretikulozyteninkubationszeit.

10. Probenanalysegerät nach Anspruch 9, **dadurch gekennzeichnet, dass**
die Steuervorrichtung (40) des Weiteren ausgestaltet ist zum Steuern der Inkubationszeit der zu testenden Probenflüssigkeit in der Probenvorbereitungsvorrichtung (20) basierend auf der dritten Zielretikulozyteninkubationszeit in Reaktion auf eine Bestätigungsanweisung des Benutzers für die dritte Zielretikulozyteninkubationszeit, um ein anderes Testergebnis der Retikulozyten in der zu testenden Probenflüssigkeit zu erhalten.

11. Probenanalysegerät nach Anspruch 1, **dadurch gekennzeichnet, dass** das Probenanalysegerät des Weiteren eine Mensch-Computer-Interaktionsvorrichtung (50) umfasst, die kommunikativ mit der Steuervorrichtung (40) verbunden ist, wobei
die Steuervorrichtung (40) des Weiteren ausgestaltet ist zum Anzeigen einer Inkubationszeitanpassungsschnittstelle in Reaktion auf eine Färbungsabnormalitätsanweisung, die durch den Benutzer basierend auf der Mensch-Computer-Interaktionsvorrichtung (50) eingegeben wurde, wobei die Inkubationszeitanpassungsschnittstelle eine Vielzahl von Retikulozyteninkubationszeiten entsprechend dem Tiertyp des Zieltiers umfasst, und wobei mindestens eine von der Vielzahl der Retikulozyteninkubationszeiten länger als die erste Zielretikulozyteninkubationszeit ist.

12. Probenanalysegerät nach Anspruch 11, **dadurch gekennzeichnet, dass** die Steuervorrichtung (40) des Weiteren ausgestaltet ist zum:
Steuern in Reaktion auf eine Anweisung des Benutzers zum Auswählen einer dritten Zielretikulozyteninkubationszeit aus der Vielzahl der Retikulozyteninkubationszeiten, wobei die dritte Zielretikulozyteninkubationszeit länger als die erste Zielretikulozyteninkubationszeit ist, und Steuern der Inkubationszeit der zu testenden Probenflüssigkeit in der Probenvorbereitungsvorrichtung (20) basierend auf der dritten Zielretikulozyteninkubationszeit, um ein anderes Testergebnis der Retikulozyten in der zu testenden Probenflüssigkeit zu erhalten.

13. Tierretikulozytentestverfahren, wobei das Verfahren umfasst:
Steuern (601) einer Probenahmevorrichtung zum Gewinnen einer Blutprobe eines Zieltiers;
Bestimmen (602) eines Tiertyps des Zieltiers, und Bestimmen einer ersten Zielretikulozyteninkubationszeit gemäß dem Zieltier basierend auf dem Tiertyp des Zieltiers, um so eine Fragmentierung von Retikulozyten in der zu testenden Probenflüssigkeit zu reduzieren;
Bestimmen (603) einer Inkubationszeit der zu testenden Probenflüssigkeit basierend auf der ersten Zielretikulozyteninkubationszeit, wobei die Inkubationszeit Reaktionsdauer der zu testenden Probenflüssigkeit und/oder Transferdauer des Transferierens der zu testenden Probenflüssigkeit zu einer Testvorrichtung umfasst; und
Steuern (604) des Reagierens der Blutprobe mit einem Behandlungsreagenz, das mit Retikulozytentests in Beziehung steht, basierend auf der Inkubationszeit, um eine zu testende Probenflüssigkeit vorzubereiten, und Steuern der zu testenden Probenflüssigkeit, so dass sie zu der Testvorrichtung transferiert wird, wobei das Behandlungsreagenz ein Retikulozytenverdünnungsmittel umfasst, und ein osmotischer Druck des Retikulozytenverdünnungsmittels niedriger als ein osmotischer Druck der Retikulozyten in der zu testenden Probenflüssigkeit ist; und
Steuern (605) der Testvorrichtung zum Testen der zu testenden Probenflüssigkeit, um ein optisches Pulssignal von Zellen in der zu testenden Probenflüssigkeit zu erhalten, und Erhalten eines Testergebnisses der Retikulozyten in der zu testenden Probenflüssigkeit basierend auf dem optischen Pulssignal der Zellen in der zu testenden Probenflüssigkeit.

14. Tierretikulozytentestverfahren nach Anspruch 13, **dadurch gekennzeichnet, dass** das Verfahren des Weiteren umfasst:
Bestimmen eines Fragmentierungsstatus der Retikulozyten in der zu testenden Probenflüssigkeit basierend auf dem Testergebnis; und
falls der Fragmentierungsstatus eine voreingestellte Bedingung erfüllt, Ausgeben von Meldungsinformationen, um eine abnormale Fragmentierungsmenge der Retikulozyten anzugeben.

15. Tierretikulozytentestverfahren nach Anspruch 14, **dadurch gekennzeichnet, dass** das Ausgeben von Meldungsinformationen, um eine abnormale Fragmentierungsmenge der Retikulozyten anzugeben, umfasst:
Anzeigen einer Testergebnisschnittstelle, wobei die Testergebnisschnittstelle die Meldungsinformationen der abnormalen Fragmentierungsmenge der Retikulozyten und eine Sprungsteuerung umfasst; und
das Verfahren des Weiteren umfasst:
Anzeigen einer Inkubationszeitanpassungsschnittstelle in Reaktion auf eine Berührungsanweisung des Benutzers auf der Sprungsteuerung, wobei die Inkubationszeitanpassungsschnittstelle eine Vielzahl von Retikulozyteninkubationszeiten entsprechend dem Tiertyp des Zieltiers umfasst, wobei mindestens eine von der Vielzahl der Retikulozyteninkubationszeiten kürzer als die erste Zielretikulozyteninkubationszeit ist.

## Revendications

1. Analyseur d'échantillon, l'analyseur d'échantillon comprenant :
un appareil d'échantillonnage (10) configuré pour collecter un échantillon de sang d'un animal cible et transférer l'échantillon de sang à un appareil de préparation d'échantillon (20) ;
l'appareil de préparation d'échantillon (20) présentant une cellule de réaction et une partie de transfert, la cellule de réaction étant configurée pour fournir un site de réaction pour l'échantillon de sang pour réagir avec un réactif de traitement lié à un test de réticulocytes, pour préparer un fluide d'échantillon à tester, et la partie de transfert étant configurée pour transférer le fluide d'échantillon à tester dans la cellule de réaction à un appareil de test (30), le réactif de traitement comprenant un diluant de réticulocytes, et une pression osmotique du diluant de réticulocytes étant inférieure à une pression osmotique de réticulocytes dans le fluide d'échantillon à tester ;
l'appareil de test (30) étant configuré pour tester le fluide d'échantillon à tester, pour obtenir un signal impulsionnel optique de cellules dans le fluide d'échantillon à tester ; et
un appareil de commande (40) relié en communication à l'appareil de préparation d'échantillon (20) et à l'appareil de test (30), et configuré pour : déterminer un type d'animal de l'animal cible, et déterminer un premier temps d'incubation de réticulocytes cible correspondant à l'animal cible sur la base du type d'animal de l'animal cible, de sorte à réduire une fragmentation des réticulocytes dans le fluide d'échantillon à tester ; commander un temps d'incubation du fluide d'échantillon à tester dans l'appareil de préparation d'échantillon (20) sur la base du premier temps d'incubation de réticulocytes cible, le temps d'incubation comprenant une durée de réaction du fluide d'échantillon à tester dans la cellule de réaction et/ou une durée de transfert pour le transfert du fluide d'échantillon à tester à l'appareil de test (30) par la partie de transfert ; et obtenir, sur la base du signal impulsionnel optique des cellules dans le fluide d'échantillon à tester, un résultat de test des réticulocytes dans le fluide d'échantillon à tester.

2. Analyseur d'échantillon selon la revendication 1, **caractérisé en ce que** l'analyseur d'échantillon comprend en outre un appareil à interaction humain-ordinateur (50),
l'appareil à interaction humain-ordinateur (50) étant relié en communication à l'appareil de commande (40), et étant configuré pour recevoir des informations d'indication de type d'animal, les informations d'indication de type d'animal portant le type d'animal ; et pour envoyer les informations d'indication de type d'animal à l'appareil de commande (40) ; et
lors de la réalisation de l'étape de détermination d'un type d'animal de l'animal cible, l'appareil de commande (40) étant spécifiquement configuré pour déterminer le type d'animal porté par les informations d'indication de type d'animal en tant que type d'animal de l'animal cible.

3. Analyseur d'échantillon selon la revendication 1, **caractérisé en ce que** lors de la réalisation de l'étape de détermination d'un premier temps d'incubation de réticulocytes cible correspondant à l'animal cible sur la base du type d'animal de l'animal cible, l'appareil de commande (40) étant spécifiquement configuré pour :
déterminer un temps d'incubation de réticulocytes correspondant à l'animal type de l'animal cible à partir d'une pluralité de correspondances prédéfinies entre des temps d'incubation de réticulocytes et des types d'animaux, et utiliser le temps d'incubation de réticulocytes déterminé en tant que premier temps d'incubation de réticulocytes cible correspondant à l'animal cible.

4. Analyseur d'échantillon selon la revendication 3, **caractérisé en ce que** l'analyseur d'échantillon comprend en outre un appareil à interaction humain-ordinateur (50),
l'appareil à interaction humain-ordinateur (50) étant relié en communication à l'appareil de commande (40), et étant configuré pour recevoir des informations personnalisées, les informations personnalisées portant des temps d'incubation de réticulocytes et des types d'animaux ; et pour envoyer les informations personnalisées à l'appareil de commande (40) ; et
l'appareil de commande (40) étant en outre configuré pour déterminer les temps d'incubation de réticulocytes et les types d'animaux portés par les informations personnalisées en tant que correspondances prédéfinies entre détente incubation de réticulocytes et des types d'animaux.

5. Analyseur d'échantillon selon la revendication 1, **caractérisé en ce que** l'analyseur d'échantillon comprend en outre un appareil à interaction humain-ordinateur (50) relié en communication à l'appareil de commande (40), l'appareil de commande (40) étant en outre configuré pour :
déterminer, sur la base du résultat de test, un état de fragmentation des réticulocytes dans le fluide d'échantillon à tester ; et
si l'état de fragmentation satisfait à une condition prédéfinie, commander à l'appareil à interaction humain-ordinateur (50) de sortir des informations d'invite pour indiquer une quantité anormale de fragmentation des réticulocytes.

6. Analyseur d'échantillon selon la revendication 5, **caractérisé en ce que** l'appareil de commande (40) est en outre configuré pour :
déterminer un deuxième temps d'incubation de réticulocytes cible qui est plus court que le premier temps d'incubation de réticulocytes cible, et commander à l'appareil à interaction humain-ordinateur (50) d'afficher le deuxième temps d'incubation de réticulocytes cible lors ou après la commande à l'appareil à interaction humain-ordinateur (50) de sortir les informations d'invite indiquant la quantité anormale de fragmentation des réticulocytes.

7. Analyseur d'échantillon selon la revendication 5, **caractérisé en ce que**
l'appareil à interaction humain-ordinateur (50) est spécifiquement configuré pour afficher une interface de résultats de test, l'interface de résultats de test comprenant les informations d'invite pour la quantité anormale de fragmentation des réticulocytes et une commande de saut ; et
l'appareil de commande (40) étant en outre configuré pour commander, en réponse à une instruction tactile de l'utilisateur pour la commande de saut, à l'appareil à interaction humain-ordinateur (50) d'afficher une interface d'ajustement de temps d'incubation, l'interface d'ajustement de temps d'incubation comprenant une pluralité de temps d'incubation de réticulocytes correspondant au type d'animal de l'animal cible, au moins l'un de la pluralité de temps d'incubation de réticulocytes étant plus court que le premier temps d'incubation de réticulocytes cible.

8. Analyseur d'échantillon selon la revendication 7, **caractérisé en ce que** l'appareil de commande (40) est en outre configuré pour :
commander, en réponse à une instruction de l'utilisateur de choisir un deuxième temps d'incubation de réticulocytes cible parmi la pluralité de temps d'incubation de réticulocytes, le temps d'incubation du fluide d'échantillon à tester dans l'appareil de préparation d'échantillon (20) sur la base du deuxième temps d'incubation de réticulocytes cible, pour obtenir un autre résultat de test des réticulocytes dans le fluide d'échantillon à tester, le deuxième temps d'incubation de réticulocytes cible étant plus court que le premier temps d'incubation de réticulocytes cible.

9. Analyseur d'échantillon selon la revendication 1, **caractérisé en ce que** l'analyseur d'échantillon comprend un appareil à interaction humain-ordinateur (50) relié en communication à l'appareil de commande (40),
l'appareil de commande (40) étant en outre configuré pour déterminer, en réponse à une instruction d'anormalité de coloration entrée par l'utilisateur sur la base de l'appareil à interaction humain-ordinateur (50), un troisième temps d'incubation de réticulocytes cible qui est plus long que le premier temps d'incubation de réticulocytes cible, et pour commander à l'appareil à interaction humain-ordinateur (50) d'afficher le troisième temps d'incubation de réticulocytes cible.

10. Analyseur d'échantillon selon la revendication 9, **caractérisé en ce que**
l'appareil de commande (40) est en outre configuré pour commander, en réponse à une instruction de confirmation de l'utilisateur pour le troisième temps d'incubation de réticulocytes cible, le temps d'incubation du fluide d'échantillon à tester dans l'appareil de préparation d'échantillon (20) sur la base du troisième temps d'incubation de réticulocytes cible, pour obtenir un autre résultat de test des réticulocytes du flux d'échantillon à tester.

11. Analyseur d'échantillon selon la revendication 1, **caractérisé en ce que** l'analyseur d'échantillon comprend en outre un appareil à interaction humain-ordinateur (50) relié en communication à l'appareil de commande (40),
l'appareil de commande (40) étant en outre configuré pour afficher une interface d'ajustement de temps d'incubation en réponse à une instruction d'anormalité de coloration entrée par l'utilisateur sur la base de l'appareil à interaction humain-ordinateur (50), l'interface d'ajustement de temps d'incubation comprenant une pluralité de temps d'incubation de réticulocytes correspondant au type d'animal de l'animal cible, et au moins l'un de la pluralité de temps d'incubation de réticulocytes étant plus long que le premier temps d'incubation de réticulocytes cible.

12. Analyseur d'échantillon selon la revendication 11, **caractérisé en ce que** l'appareil de commande (40) est en outre configuré pour :
commander, en réponse à une instruction de l'utilisateur pour choisir un troisième temps d'incubation de réticulocytes cible parmi la pluralité de temps d'incubation de réticulocytes, le troisième temps d'incubation de réticulocytes cible étant plus long que le premier temps d'incubation de réticulocytes cible, et commander le temps d'incubation du fluide d'échantillon à tester dans l'appareil de préparation d'échantillon (20) sur la base du troisième temps d'incubation de réticulocytes cible, pour obtenir un autre résultat de test des réticulocytes dans le fluide d'échantillon à tester.

13. Procédé de test de réticulocytes d'animaux, le procédé comprenant :
la commande (601) d'un appareil d'échantillonnage pour collecter un échantillon de sang d'un animal cible ;
la détermination (602) d'un type d'animal de l'animal cible, et la détermination d'un premier temps d'incubation de réticulocytes cible correspondant à l'animal cible sur la base du type d'animal de l'animal cible, de sorte à réduire une fragmentation de réticulocytes dans le flux d'échantillon à tester ;
la détermination (603), sur la base du premier temps d'incubation de réticulocytes cible, d'un temps d'incubation du fluide d'échantillon à tester, le temps d'incubation comprenant une durée de réaction du fluide d'échantillon à tester et/ou une durée de transfert pour transférer le fluide d'échantillon à tester à un appareil de test ; et
la commande (604), sur la base du temps d'incubation, à l'échantillon de sang de réagir avec un réactif de traitement lié à un test de réticulocytes, pour préparer un fluide d'échantillon à tester, et la commande au fluide d'échantillon à tester d'être transféré à l'appareil de test, le réactif de traitement comprenant un diluant de réticulocytes, et une pression osmotique du diluant de réticulocytes étant inférieure à une pression osmotique des réticulocytes dans le fluide d'échantillon à tester ; et
la commande (605) à l'appareil de test de tester le fluide d'échantillon à tester, pour obtenir un signal impulsionnel optique de cellules dans le fluide d'échantillon à tester, et l'obtention, sur la base du signal impulsionnel optique des cellules dans le fluide d'échantillon à tester, d'un résultat de test des réticulocytes dans le fluide d'échantillon à tester.

14. Procédé de test de réticulocytes d'animaux selon la revendication 13, **caractérisé en ce que** le procédé comprend en outre :
la détermination, sur la base du résultat de test, d'un état de fragmentation des réticulocytes dans le fluide d'échantillon à tester ; et
si l'état de fragmentation satisfait à une condition prédéfinie, la sortie d'informations d'invite pour indiquer une quantité anormale de fragmentation des réticulocytes.

15. Procédé de test de réticulocytes d'animaux selon la revendication 14, **caractérisé en ce que** la sortie d'informations d'invite pour indiquer une quantité anormale de fragmentation des réticulocytes comprend : l'affichage d'une interface de résultats de test, l'interface de résultats de test comprenant les informations d'invite de la quantité anormale de fragmentation des réticulocytes et une commande de saut ; et
le procédé comprenant en outre :
l'affichage d'une interface d'ajustement de temps d'incubation en réponse à une instruction tactile de l'utilisateur pour la commande de saut, l'interface d'ajustement de temps d'incubation comprenant une pluralité de temps d'incubation de réticulocytes correspondant au type d'animal de l'animal cible, au moins l'un de la pluralité de temps d'incubation de réticulocytes étant plus court que le premier temps d'incubation de réticulocytes cible.
